# EUROPEAN PATENT SPECIFICATION

(11) **EP 0 444 000 B1**
(45) Date of publication and mention of the grant of the patent: **12.01.1994**
(21) Application number: 91850031.5
(22) Date of filing: 12.02.1991
(51) Int. Cl.: A61K 31/70, A61K 9/48, A61K 47/02, A61K 47/22

(54) **Storage-stable glucosamine sulphate oral dosage forms and methods for their manufacture**
Lagerstabile orale Glucosaminsulfat-Dosisformen und Verfahren zu deren Herstellung
Formes de doses orales du sulfate de la glucosamine stables au stockage et leurs procédés de préparation

(30) Priority: 22.02.1990 US 483572
(43) Date of publication of application: 28.08.1991
(73) Proprietor: Health Maintenance Programs, Inc., Elmsford, New York 10523 (US)
(72) Inventor: Demopoulos, Harry Byron, Scarsdale, N.Y. 10583 (US)
(74) Representative: Kristiansen, Alf P.

(56) References cited:
- EP-A- 0 178 602
- EP-A- 0 214 642
- EP-A- 0 264 259
- US-A- 4 642 237

## Description

The present invention relates to the field of storage-stable glucosamine sulphate oral dosage forms and methods for their manufacture.

Glucosamine sulphate is a well known and extremely important substance in the treatment of rheumatic fever, arthritic and arthrosic complaints, both in the acute and chronic forms, and, generally, of all pathological conditions originating from metabolic disorders of the osteo-articular tissue. See U.S. Patent No. 3,683,076, and United Kingdom Patent No. 1,056,331. See also I. Setnikar et al., "Pharmacokinetics of Glucosamine in the Dog and in Man," Arzneimittelforschung, April 1986, 36(4) pp. 729-35.

Glucosamine, used in the form of the salt with hydrochloric, sulphuric, phosphoric, or other biocompatible acids, is known to have an anti-inflammatory effect when taken orally or parenterally. Tapadinhas et al., *Pharmatherapeutica,* 3(3), 157-168 (1982).

The synthesis of glucosamine sulphate was described by Breuer in 1898 (Chem. Ber. 31, 2197) and an industrial method is the subject of U.K. Patent No. 1,056,331. U.S. Patent No. 3,683,076 and Swiss Patent No. 525,861.

However, glucosamine sulphate has several properties which render its packaging problematic. Specifically, glucosamine sulphate is highly hygroscopic in nature and the amino group oxidizes readily.

These unfavorable properties give rise to difficulties and limitations in the practical use of glucosamine sulphate. For example, oral forms, such as tablets or capsules, require anti-oxidants, such as sodium hyposulphite, to be present in their formulations. Generally, these substances, although blocking the oxidation of the amino group, do not solve the problem of the hygroscopic nature of the sulphate. This necessitates the preparation of these forms in environments with a relative humidity not greater than 30%, and even then, the results are unsatisfactory. Similar comments apply to rectal forms (suppositories) which, even if kept under dry, refrigerated conditions, degrade with considerable rapidity.

U.S. Patent No. 4,642,340 suggests that it is possible to stabilize glucosamine sulphate by the formation of a mixed salt thereof with sodium chloride.

Published European Application EP 214, 642, published March 10, 1987, also suggests the use of a stabilizing salt, but discloses the use of MgCl₂, NaBr and KBr instead of NaCl.

Furthermore, it is known that ascorbic acid may be used to prevent the oxidation of materials such as compositions including cyclo-(N-methyl-Ala-Tyr-D-Trp-Lys-Val-Phe) and a selected non-steroidal anti-inflammatory drug. See U.S. Patent No. 4,474,766.

U.S. Patent No. 4,590,067 relates to a treatment of periodontal disease and describes a composition for topical applications, including a calcium source, glucosamine or other simple sugars; or amino sugar having anti-inflammatory activity, ascorbic acid and tyrosine, phenylalanine or other precursor or stimulant of epinephrine or nor-epinephrine production. Bone meal or the equivalent serves as a source of calcium for tooth regeneration, as a general cleansing agent, and as a filler. Equivalent substances include biologically compatible calcium salts, representative of which are calcium gluconate, calcium carbonate, tricalcium and dicalcium phosphate, dolomite and the like.

The anti-inflammatory substance may be selected from the amino sugars, glucosamine, D-mannosamine, D-galactosamine, their biocompatible acid addition salts, glucosamine-6-phosphate, N-acetyl-D-glucosamine, N-acetyl-D-galactosamine, UDP-N-acetylglucosamine; the sugars 2 deoxy-D-glucose, 2-deoxy-D-galactose and mannose; and the amino acids cysteine, creatine, creatinine, L-tryprophan, valine, alanine, glycine, glutamine, aspartic acid and S-methyl-cysteine. The ascorbic acid is used to combat periodontal disease.

U.S. Patent No. 4,772,591 relates to a method for accelerated wound healing and discloses a composition very similar to that disclosed in U.S. Patent No. 4,590,067 discussed above. However, the calcium source is optional if the composition is to be given orally and the patient has sufficient dietary calcium. The ascorbic acid is used for curing periodontal disease and for certain healing properties. See also U.S. Patent No. 4,647,453 (=EP-A-0178602).

Despite the work in stabilizing glucosamine sulphate that has gone before and the therapeutic importance of this compound, there remains the need to alternate methods of stabilizing glucosamine sulphate.

The present invention provides a composition which includes glucosamine sulphate in a highly storage-stable form.

In accordance with this aspect of the present invention, there is provided a oral dosage formulation including a measured amount of glucosamine sulphate; and ascorbic acid in an amount of at least 25mg per 100mg of glucosamine sulphate and calcium carbonate in an amount of at least 35mg per 100mg of glucosamine suphate substantially homogeneously admixed together and having enhanced storage stability.

Also in accordance with this aspect of the present invention, there is provided a storage-stable oral dosage formulation including a measured amount of glucosamine sulphate; an ascorbic acid selected from the group consisting of crystalline ascorbic acid or a physiologically acceptable crystalline ascorbate salt, and calcium carbonate the ascorbic acid and the calcium carbonate being present in an amount effective to stabilize the measured amount of the glucosamine sulphate, substantially homogeneously admixed therewith.

The formulation thus produced has several advantages. First, it has enhanced storage stability and adequately addresses both the hygroscopic and oxidative tendencies of glucosamine sulphate. While not wishing to be bound by any particular theory of operability, it is believed that the ascorbic acid acts as an antioxidant and the carbonate acts as a desiccant.

In a particularly preferred aspect of the present invention, the formulation includes either crystalline ascorbic acid or a physiologically acceptable crystalline ascorbate salt. The use of this form of ascorbic acid provides additional lubricating properties which allow for the manufacture of an oral dosage form without the need to resort to additional tableting adjuvants. This allows for a reduced production cost, eliminates the need to employ ingredients which may adversely affect the health of a patient to which they are administered, and reduces the possibility of reactions between ingredients which may adversely effect the storage stability of the glucosamine sulphate. Also, the unique combination of ingredients of this invention provides a safe system for the patient, as the calcium carbonate tends to neutralize the ascorbic acid when an oral dosage form of the present invention is ingested.

It is also an object of the present invention to provide a method of stabilizing an oral dosage form of glucosamine sulphate that results in a product of enhanced storage stability.

In accordance with this aspect of the present invention, there is provided a method of stabilizing an oral dosage form of glucosamine sulphate which includes the steps of providing a measured amount of glucosamine sulphate, admixing, substantially homogeneously therewith, ascorbic acid and calcium carbonate in amounts effective to stabilize the measured amount of the glucosamine sulphate; and forming the admixture into an oral dosage form, having enhanced storage stability.

Also in accordance with this aspect of the present invention, there is provided a method of stabilizing an oral dosage from of glucosamine sulphate including the steps of providing a measured amount of glucosamine sulphate, admixing, substantially homogeneously therewith, ascorbic acid selected from the group consisting of crystalline ascorbic acid and a physiologically acceptable crystalline ascorbate salt, and calcium carbonate said ascorbic acid on said calcium carbonate being provided in an amount effective to stabilize the measured amount of the glucosamine sulphate; and forming the admixture into an oral dosage form, having enhanced storage stability.

The method provides a protocol by which glucosamine sulphate containing oral dosage forms having enhanced storage stability may be manufactured.

As previously noted, glucosamine sulphate synthesis was described by Breuer in 1898 (Chem. Ber. 31, 2197) and an industrial method is described in U.K. Patent No. 1,056,331, U.S. Patent No. 3,683,076 and Swiss Patent No. 525,861. Glucosamine sulphate prepared in accordance with Swiss Patent No. 525,861 generally involves liberation of the glucosamine from its hydrochloride in an aqueous-alcoholic medium, in the presence of triethylamine, and subsequent treatment of the obtained base in an acetone medium with the stoichiometric quantity of concentrated sulphuric acid to give the glucosamine sulphate. In the Swiss patent cited above, ethyl ether is used, but in industrial preparations on a large scale, it is preferred to use acetone which is much less dangerous and moreover, yields a product with the same characteristics as those described in the Swiss patent.

Ascorbic acid (Vitamin C) can be obtained in reduced form from a number of commercial sources. While the present invention includes the use of commercial ascorbic acid in formulating the storage-stable dosage formulations of the present invention, it should be recognized that conventional dry powder Vitamin C formulations have certain limitations. For example, the flow characteristics of two such products are impaired because of the recognized hygroscopicity of ascorbic acid (see, for example, Pipher U.S. Patent No. 2,846,353, at column 2, lines 21-27). In addition, it has long been known that ascorbic acid is a reducing agent which, upon oxidation (e.g., during storage at elevated temperatures), is susceptible to discoloration (see, for example, Magid U.S. Patent No. 3,493,659, at column 1, lines 21-23). For these reasons, the preferred formulation in accordance with the present invention includes the use of crystalline ascorbic acid or crystalline physiologically acceptable ascorbate salts.

The crystalline ascorbic acid materials referred to herein include both ascorbic acid per se, and the physiologically acceptable cationic ascorbate salts, e.g., sodium ascorbate, calcium ascorbate, and magnesium ascorbate. The crystalline ascorbic acid materials are generally rhomboid shaped, plate-like crystals which may have particle sizes passing anywhere from 200 to 10 mesh screens, with crystals of medium-fine grade (30-80 mesh) being particularly preferred. The crystals may be directly incorporated in the dry powder formulations of the present invention.

The crystalline ascorbic acid materials utilized herein are commercially available substances which may be produced by the hydrogenation of D-glucose to D-sorbitol, followed by the microbial oxidation to L-sorbose, carboxylation to diacetone-2-keto-L-galonic acid, and conversion to ascorbic acid by heating with hydrogen chloride. The ascorbate salts may of course be produced directly from the acid if desired.

The structure of the crystalline ascorbic acid materials utilized in accordance herewith has been elucidated in the literature (see "The Crystal Structure of L-Ascorbic Acid 'Vitamin C'", J. Hvoslef, Acta Chemica Scandinavica, 18, No. 3, 1964, pp. 841-842). It has thus been shown by Fourier mapping that crystalline ascorbic acid possesses the following, generally accepted structural formula: 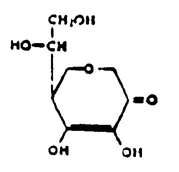
The 5-member ring is substantially planar. The material has a monoclinic crystalline structure, usually in plate-like form with occasional needles, four molecules of the ascorbic acid defining a unit cell. Two unit cells form pairs in pseudo-symmetrical order, whereby the molecules as well as the unit cells are connected by hydrogen bonding. The resultant planar crystals are thus slidable relative to each other, permitting them to act as a dry lubricant to impart free flowability to powders which otherwise tend to cake, stick or clump during encapsulation or tableting thereof.

In addition, the individual ascorbic acid (or ascorbate) crystals are readily fracturable due to the lack of extreme density and strength within the individual crystal structures. It is thus possible to break and compact the individual crystals upon subjecting the dry powder formulations containing the same to compressive forces during encapsulation or tableting. The dry powder formulation incorporating the crystalline ascorbic acid materials are thus readily compressible, as well as free flowing.

It is further believed that the crystalline synthetic ascorbic acid materials utilized in accordance with the present invention are more stable than natural ascorbic acid crystals or the amorphous, fine powder ascorbic acid utilized in conventional multi-vitamin formulations. Materials of these latter types have been described as being subject to oxidation upon exposure to air and light (see the Merck Index, 8th Edition, 1968, p. 105). The synthetic crystalline ascorbic acid utilized in the formulations hereof may, because of the existence of hydrogen bonding in the crystal, be less susceptible to autoxidation to dehydro-ascorbic acid and thus exhibit superior stability.

The incorporation of crystalline ascorbic acid into dry powder formulations having improved flow and compressibility characteristics was described in U.S. Patent No. 4,454,125.

According to The Merck Index, 9th edition, calcium carbonate exists in nature and the minerals aragonite, calcite and vaterite. Calcium carbonate is generally an odorless, tasteless powder or crystal which is practically insoluble in water. Calcium carbonate is commercially available from a number of sources.

The amount of calcium carbonate and ascorbic acid used in accordance with the present invention depends upon the amount of glucosamine sulphate to be delivered by the individual oral dosage. In all cases, there should be an amount of ascorbic acid and calcium carbonate sufficient to be effective to stabilize the measured amount of glucosamine sulphate to be delivered by the dosage. While not wishing to be bound by any particular theory of operation, it is believed that the ascorbic acid should be present in an amount effective to prevent the oxidation of the glucosamine sulphate and the calcium carbonate should be present in an amount effective to dessicate the glucosamine sulphate. If a conventional dry powder ascorbic acid is used in the practice of the present invention, it is advisable to use additional calcium carbonate to compensate for the hygroscopic properties thereof.

The amount of glucosamine sulphate used in the practice of the present invention depends upon the reason for prescribing and/or dispensing thereof. Typical single oral dosages could include from between 50 mg to 1500 mg. In preferred formulations, between 100 mg and 1000 mg may be used. In a more preferred embodiment of the present invention between 200 mg and 500 mg are used. Most preferably, the formulation in accordance with the present invention includes about 400 mg of glucosamine sulphate.

The amount of ascorbic acid used in accordance with the present invention should be at least 25 mg per 100 mg of glucosamine sulphate. In a preferred embodiment of the present invention, the amount of ascorbic acid should be between 25 mg and 100 mg and more preferably about 75 mg per 100 mg of glucosamine sulphate.

The amount of calcium carbonate used in accordance with the present invention should be at least 35 mg per 100 mg of glucosamine sulphate. In a preferred embodiment in accordance with the present invention, the amount of calcium carbonate should be between about 37 mg and about 100 mg and more preferably about 75 mg per 100 mg of glucosamine sulphate.

It may be acceptable to add tableting adjuvants to help the flowability of the materials, their compaction, color, taste, binding and the like. However, the inclusion of these ingredients should be limited to those which will not adversely effect the health of the patient to which they are administered or complicate the stabilization of the glucosamine sulphate.

Conventional tableting adjuvants and the conventional amounts used are described in Lieberman et al., Pharmaceutical Dosage Forms-Tablets, volume 1, 2d Ed., published by Marcel Dekker, Inc., in 1989. Tableting adjuvants clearly do not include other "active" ingredients such as the amino acids described in the 4,772,591 patent discussed previously.

In a preferred embodiment in accordance with the present invention, the use of crystalline ascorbic acid or a physiologically acceptable crystalline ascorbic salt in the formulation eliminates the need for such adjuvants.

The present invention includes the realization that the stabilization of glucosamine sulphate will require a compound solution involving a plurality of ingredients working in concert. The invention also includes an understanding of the nature of the instability of glucosamine sulphate and the development of a protocol and formulation whereby stability may be enhanced. By the practice of the present invention, the shelf life of glucosamine sulphate capsules may be extended from about three months to an expected 1 to 3 years. This allows for large scale production to be commercially feasible.

The method in accordance with the present invention involves providing a measured amount of glucosamine sulphate and homogeneously admixing therewith, an appropriate amount of stabilizing ingredients. Thus, once the amount of glucosamine sulphate to be delivered in each dosage has been determined and a sufficient amount of glucosamine sulphate raw material has been provided to accommodate the desirable number of dosage units to be produced, an effective amount of ascorbic acid and calcium carbonate are admixed substantially homogeneously therewith.

The term "effective amount" means enough ascorbic acid and calcium carbonate to effectively stabilize the measured amount of the glucosamine sulphate.

The mixture is then formed into one, or a plurality of individual oral dosage forms including capsule, tablets, suspensions and the like, having enhanced storage stability.

In a most preferred embodiment in accordance with the present invention, the oral dosage form is a capsule.

The foregoing will be better understood with reference to the following example. The example is for purposes of illustration and is not to be considered limiting as to the scope and nature of the present invention.

### EXAMPLE I

22.857 kg of glucosamine sulphate (Pfansftiehl Labs, Inc. of Waukegan, Ill, Lot No. 19470A) 17.142 Kg of crystalline ascorbic acid (Hoffman-LaRoche, Nutley, NJ Lot No. 276119) and 17.142 kg of extra heavy precipitated calcium carbonate (White Pigment Corp., Florence, VT, Lot No. 9257) were used. The glucosamine sulphate was added to a 283 l (10 cu.ft). Patterson-Kelly twin shell tumble blender and tumbled for approximately 10 min. to break up any agglomerates. The planar crystalline ascorbic acid is then added and the two components are mixed for approximately an additional 20 min. The extra heavy precipitated calcium carbonate is then added and mixing is conducted for approximately an additional 45 min. The resulting admixture was loaded into the powder hopper of a Zanasi AZ-20 intermittent motion capsule filling machine in batches of 20 kg. The 6 powder dosators and pistons were treated with a 3/1000 mm chrome/nickel/boron coating and polished with diamond grit by Isometric Microfinishing Co. in Edison, NJ for increased lubricity. Dosators are set at 2.1 cm, powder pan set at 5.5 cm and dosator compression set at "000". Room humidity must be kept between 30-45% at 17-19°C (62-66°F). Capsules are filled at a rate of 14,300 per hour to a fill weight of 1.0 g into 2 piece hard gelatin capsules of size "00" from Pharmaphil Corp., Windsor, Canada.

## Claims

1. A method of stabilizing an oral dosage form of glucosamine sulphate, characterized by admixing, substantially homogeneously with said glucosamine sulphate, ascorbic acid in an amount of at least 25 mg per 100 mg of glucosamine sulphate and calcium carbonate in an amount of at least 35 mg per 100 mg of glucosamine sulphate so as to form said oral dosage form having enhanced storage stability.

2. The method of stabilizing an oral dosage form of glucosamine sulphate according to Claim 1, characterized by said ascorbic acid being present in an amount of between 25 mg and 100 mg per 100 mg of said glucosamine sulphate.

3. The method of stabilizing an oral dosage form of glucosamine sulphate according to Claim 2, characterized by said ascorbic acid being present in an amount of 75 mg per 100 mg of said glucosamine sulphate.

4. The method of stabilizing an oral dosage form of glucosamine sulphate according to Claims 1-3, characterized by said ascorbic acid being crystalline ascorbic acid or a physiologically acceptable crystalline ascorbate salt.

5. The method of stabilizing an oral dosage form of glucosamine sulphate according to Claim 1, characterized by said calcium carbonate being present in an amount of between 37 mg and 100 mg per 100 mg of glucosamine sulphate.

6. The method of stabilizing an oral dosage form of glucosamine sulphate according to Claim 5, characterized by said calcium carbonate being present in an amount of 75 mg per 100 mg of glucosamine sulphate.

7. A storage-stable oral dosage formulation of glucosamine sulphate characterized by a homogeneous admixture of ascorbic acid in an amount of at least 75 mg per 100 mg of glucosamine sulphate and calcium carbonate in an amount of at least 35 mg per 100 mg of glucosamine sulphate.

8. The storage-stable oral dosage formulation according to Claim 7, characterized by said ascorbic acid being present in an amount of between 25 mg and 100 mg per 100 mg of said glucosamine sulphate.

9. The storage-stable oral dosage formulation according to Claim 8, characterized by said ascorbic acid being present in an amount of 75 mg per 100 mg of glucosamine sulphate.

10. The storage-stable oral dosage formulation according to Claims 7-9, characterized by said ascorbic acid being crystalline ascorbic acid or a physiologically acceptable crystalline ascorbate salt.

11. The storage-stable oral dosage formulation according to Claim 7, characterized by said calcium carbonate being present in an amount of between 37 mg and 100 mg per 100 mg of said glucosamine sulphate.

12. The storage-stable oral dosage formulation according to Claim 11, characterized by said calcium carbonate being present in an amount of 75 mg per 100 mg of said glucosamine sulphate.

## Patentansprüche

1. Verfahren zur Stabilisierung einer oralen Verabreichungsform von Glucosaminsulfat, dadurch gekennzeichnet, daß man mit dem Glucosaminsulfat Ascorbinsäure in einer Menge von mindestens 25 mg pro 100 mg Glucosaminsulfat und Calciumcarbonat in einer Menge von mindestens 35 mg pro 100 mg Glucosaminsulfat im wesentlichen homogen vermischt, um eine orale Verabreichungsform mit erhöhter Lagerstabilität auszubilden.

2. Verfahren zur Stabilisierung einer oralen Verabreichungsform von Glucosaminsulfat gemäß Anspruch 1, dadurch gekennzeichnet, daß die Ascorbinsäure in einer Menge von zwischen 25 mg und 100 mg pro 100 mg des Glucosaminsulfats vorhanden ist.

3. Verfahren zur Stabilisierung einer oralen Verabreichungsform von Glucosaminsulfat gemäß Anspruch 2, dadurch gekennzeichnet, daß die Ascorbinsäure in einer Menge von 75 mg pro 100 mg des Glucosaminsulfats vorhanden ist.

4. Verfahren zur Stabilisierung einer oralen Verabreichunsform von Glucosaminsulfat gemäß einem der Ansprüche 1 bis 3, dadurch gekennzeichnet, daß die Ascorbinsäure eine kristalline Ascorbinsäure oder ein physiologisch annehmbares kristallines Ascorbinsäuresalz ist.

5. Verfahren zur Stabilisierung einer oralen Verabreichungsform von Glucosaminsulfat gemäß Anspruch 1, dadurch gekennzeichnet, daß das Calciumcarbonat in einer Menge von zwischen 37 mg und 100 mg pro 100 mg Glucosaminsulfat vorhanden ist.

6. Verfahren zur Stabilisierung einer oralen Verabreichungsform von Glucosaminsulfat gemäß Anspruch 5, dadurch gekennzeichnet, daß das Calciumcarbonat in einer Menge von 75 mg pro 100 mg Glucosaminsulfat vorhanden ist.

7. Lagerstabile Formulierung von Glucosaminsulfat zur oralen Verabreichung, dadurch gekennzeichnet, daß sie eine homogene Mischung mit Ascorbinsäure in einer Menge von mindestens 25 mg pro 100 mg Glucosaminsulfat und Calciumcarbonat in einer Menge von mindestens 35 mg pro 100 mg Glucosaminsulfat ist.

8. Lagerstabile Formulierung zur oralen Verabreichung gemäß Anspruch 7, dadurch gekennzeichnet, daß die Ascorbinsäure in einer Menge von zwischen 25 mg und 100 mg pro 100 mg Glucosaminsulfat vorhanden ist.

9. Lagerstabile Formulierung zur oralen Verabreichung gemäß Anspruch 8, dadurch gekennzeichnet, daß die Ascorbinsäure in einer Menge von 75 mg pro 100 mg Glucosaminsulfat vorhanden ist.

10. Lagerstabile Formulierung zur oralen Verabreichung gemäß einem der Ansprüche 7 bis 9, dadurch gekennzeichnet, daß die Ascorbinsäure kristalline Ascorbinsäure oder ein physiologisch annehmbares kristallines Ascorbinsauresalz ist.

11. Lagerstabile Formulierung zur oralen Verabreichung gemäß Anspruch 7, dadurch gekennzeichnet, daß das Calciumcarbonat in einer Menge zwischen 37 mg und 100 mg pro 100 mg Glucosaminsulfat vorhanden ist.

12. Lagerstabile Formulierung zur oralen Verabreichung gemäß Anspruch 11, dadurch gekennzeichnet, daß das Calciumcarbonat in einer Menge von 75 mg pro 100 mg Glucosaminsulfat vorhanden ist.

## Revendications

1. Procédé de stabilisation d'une forme pour dose orale de sulfate de glucosamine, caractérisé en ce qu'il consiste à mélanger d'une manière sensiblement homogène avec ledit sulfate de glucosamine, de l'acide ascorbique selon une quantité au moins égale à 25 mg pour 100 mg de sulfate de glucosamine et du carbonate de calcium selon une quantité au moins égale à 35 mg pour 100 mg de sulfate de glucosamine, de manière à constituer une forme pour dose orale ayant une stabilité au stockage renforcée.

2. Procédé de stabilisation d'une forme pour dose orale de sulfate de glucosamine selon la revendication 1, caractérisé en ce que la quantité d'acide ascorbique est comprise entre 25 mg et 100 mg pour 100 mg dudit sulfate de glucosamine.

3. Procédé de stabilisation d'une forme pour dose orale de sulfate de glucosamine selon la revendication 2, caractérisé en ce que la quantité d'acide ascorbique est de 75 mg pour 100 mg dudit sulfate de glucosamine.

4. Procédé de stabilisation d'une forme pour dose orale de sulfate de glucosamine selon les revendications 1 à 3, caractérisé en ce que l'acide ascorbique est un acide ascorbique cristallin ou un sel cristallin d'ascorbate physiologiquement acceptable.

5. Procédé de stabilisation d'une forme pour dose orale de sulfate de glucosamine selon la revendication 1, caractérisé en ce que la quantité de carbonate de calcium est comprise entre 37 mg et 100 mg pour 100 mg de sulfate de glucosamine.

6. Procédé de stabilisation d'une forme pour dose orale de sulfate de glucosamine selon la revendication 5, caractérisé en ce que la quantité de carbonate de calcium est de 75 mg pour 100 mg de sulfate de glucosamine.

7. Formulation d'une dose orale de sulfate de glucosamine stable au stockage, caractérisée en ce qu'elle comprend un mélange homogène d'acide ascorbique suivant une quantité d'au moins 25 mg pour 100 mg de sulfate de glucosamine, et de carbonate de calcium suivant une quantité d'au moins 35 mg pour 100 mg de sulfate de glucosamine.

8. Formulation d'une dose orale stable au stockage selon la revendication 7, caractérisée en ce que la quantité d'acide ascorbique est comprise entre 25 mg et 100 mg pour 100 mg de sulfate de glucosamine.

9. Formulation d'une dose orale stable au stockage selon la revendication 8, caractérisée en ce que la quantité d'acide ascorbique est de 75 mg pour 100 mg de sulfate de glucosamine.

10. Formulation d'une dose orale stable au stockage selon les revendications 7 à 9, caractérisée en ce que l'acide ascorbique est un acide ascorbique cristallin ou un sel cristallin d'ascorbate physiologiquement acceptable.

11. Formulation d'une dose orale stable au stockage selon la revendication 7, caractérisée en ce que la quantité de carbonate de calcium est comprise entre 37 mg et 100 mg pour 100 mg de sulfate de glucosamine.

12. Formulation d'une dose orale stable au stockage selon la revendication 11, caractérisée en ce que la quantité de carbonate de calcium est de 75 mg pour 100 mg de sulfate de glucosamine.
